# EUROPÄISCHE PATENTSCHRIFT

(11) **EP 1 486 787 B1**
(45) Veröffentlichungstag und Bekanntmachung des Hinweises auf die Patenterteilung: **26.01.2011**
(21) Anmeldenummer: 04013787.9
(22) Anmeldetag: 11.06.2004
(51) Int. Cl.: G01N 33/574

(54) **Verfahren zur In-Vitro-Diagnose eines Glioms oder Astrozytoms und pharmazeutische Mischung zur Behandlung des Glioms, Astrozytoms oder eines Rezidivs davon**
Process for the in vitro diagnosis of a glioma or an astrocytoma and a pharmaceutical mixture for the treatment of a glioma or an astrocytoma or a reappearance therefrom
Procédé pour diagnostiquer un gliome ou un astrocytome in vitro et un mélange pharmaceutique pour le traîtement de gliomes, d'astrocytomes ou d'une rechute de gliome ou d'astrocytome

(30) Priorität: 12.06.2003 DE 10326545
(43) Veröffentlichungstag der Anmeldung: 15.12.2004
(73) Patentinhaber: Kübler, Ulrich, Dr., 81675 München (DE)
(72) Erfinder: Kübler, Ulrich, Dr., 81675 München (DE)
(74) Vertreter: Nätebusch, Roderich

(56) Entgegenhaltungen:
- EP-A- 0 584 715
- PFREUNDSCHUH M ET AL: "SEROLOGICAL ANALYSIS OF CELL SURFACE ANTIGENS OF MALIGNANT HUMAN BRAIN TUMORS" PROCEEDINGS OF THE NATIONAL ACADEMY OF SCIENCES OF THE UNITED STATES OF AMERICA, Bd. 75, Nr. 10, 1978, Seiten 5122-5126, XP009035992 ISSN: 0027-8424
- NAKAYAMA YOSHIYA ET AL: "Stromal angiogenesis in human glioma: A role of platelet-derived endothelial cell growth factor" SURGICAL NEUROLOGY, Bd. 49, Nr. 2, Februar 1998 (1998-02), Seiten 181-188, XP001183823 ISSN: 0090-3019
- AKIRA ITO ET AL: "Heat shock protein 70 expression induces antitumor immunity during intracellular hyperthermia using magnetite nanoparticles" CANCER IMMUNOLOGY AND IMMUNOTHERAPY, BERLIN, DE, Bd. 52, Januar 2003 (2003-01), Seiten 80-88, XP002969599 ISSN: 0340-7004
- MURPHY P ET AL: "Modulation of plasminogen activator and plasminogen activator inhibitor expression in the human U373 glioblastoma/astrocytoma cell line by inflammatory mediators." EXPERIMENTAL CELL RESEARCH. JAN 1992, Bd. 198, Nr. 1, Januar 1992 (1992-01), Seiten 93-100, XP009037424 ISSN: 0014-4827
- DATABASE MEDLINE [Online] US NATIONAL LIBRARY OF MEDICINE (NLM), BETHESDA, MD, US; November 1995 (1995-11), OKAMURA K: "[Malignant glioma]" XP002299621 Database accession no. NLM8538025 & NIPPON RINSHO. JAPANESE JOURNAL OF CLINICAL MEDICINE. NOV 1995, Bd. 53, Nr. 11, November 1995 (1995-11), Seiten 2678-2683, ISSN: 0047-1852
- DELPINO A ET AL: "CHARACTERIZATION OF A NEW HIGH-TEMPERATURE-INDUCED 66-KDA HEAT-SHOCK PROTEIN, ANTIGENICALLY RELATED TO HEAT-SHOCK PROTEIN 72" JOURNAL OF CELLULAR BIOCHEMISTRY, WILEY-LISS INC, US, Bd. 63, Nr. 1, Oktober 1996 (1996-10), Seiten 51-60, XP009035987 ISSN: 0730-2312
- KORKOLOPOULOU P ET AL: "Hypoxia-inducible factor 1alpha/vascular endothelial growth factor axis in astrocytomas. Associations with microvessel morphometry, proliferation and prognosis." NEUROPATHOLOGY AND APPLIED NEUROBIOLOGY. JUN 2004, Bd. 30, Nr. 3, Juni 2004 (2004-06), Seiten 267-278, XP001183709 ISSN: 0305-1846

## Beschreibung

Die vorliegende Erfindung bezieht sich auf ein Verfahren zur In-vitro-Diagnose eines Glioms oder Astrozytoms eines Individuums als Glioblastoma-multiforme und auf eine pharmazeutische Mischung zur Behandlung des Glioms, Astrozytoms oder eines Rezidivs davon.

Die bisher üblicherweise eingesetzten Krebsdiagnoseverfahren sind relativ ungenau bzw. sie liefern erst dann aussagekräftige Ergebnisse, wenn die jeweiligen Tumoren im Wachstum schon erheblich fortgeschritten sind, also eine beachtliche Größe aufweisen - die betreffenden Diagnoseverfahren sind mithin zu spät wirksam, um das Vorliegen eines oder mehrerer Tumoren für die Anwendung einer wirksamen, sanften Therapie zu erkennen. Dann können in der Regel nur noch chirurgische, chemotechnische und/oder radiologische Verfahren zur Entfernung der Tumoren angewandt werden. Solche Verfahren schließen allerdings nicht aus, dass sich Rezidive bilden.

so ist es bekannt (PNAS; Vol. 75, Nr. 19, 1978, S. 5122-5126, insbesondere Zusammenfassung; S.5122, Spatte 2, Abs. 2; S.5123, Spatte 1, letzter Abs. ff. ), Die detektion von Astrozytomzellen in Serum eines Patienten mit Hilfe der detektion von im Serum zirkulierrenden Anti-körpern vorzunehmen, die mit Zelloberflächenantigen von kultivierten autologen Astrozytomzellen reagieren.

Es ist ferner die Detektion von Glioblastomen und Astrocytomen bekannt (Surgical Neurology, vol. 49, Nr·2, 1998, S.181-188; S.181, Spatte 1, letiter Abs.), wie z.B. auch Glioblastoma multi-forme, bei der PD-ECGF- und GFAP- positive Zellen innerhalb von Blutgefäßen innerhalb oder am Rand eines soliden Tumors detektiert werden. Dies heißt, dass hier Antigen aus dem tumor-blutgefäßsystem detektiert wird, nachdem chirurgisch eingegriffen wurde, um den Tumor für die anschließende Detektion in dessen Blutgefäßen 24 entnehmen.

Es ist außerdem bekannt, die zu therapeutischen Zwecken durchgeführte Gewinnung von transformierten Zellen aus dem Blut lebender Organismen mit Hilfe einer Apherese vorzunehmen (Zusammenfassung; Ansprüche 1, 9 von EP 0 584 715 B1). Die Apherese erlaubt die quasikontinuierliche Entnahme von bestimmten Blutfraktionen in hohen Mengen, ohne den Patienten zu belasten. Auf diese weise können für den patienten individuums-spezifische Antikörper für ein Diagnostikum hergestellt werden (Seite 6, Zeilen 19-23 von EP 0584 715 B1). Astrozytome und gliome oder Glioblastome sind nicht offenbart.

Dabei kann man bei gesunden Individuen generell keinerlei maligne Zellen nach leukapheretischer Anreicherung aus dem Blutstrom des Kreislaufes erhalten oder in einer Blutprobe nachweisen.

1 bis 10 maligne, das heißt Onkoproteine exprimierende Zellen pro 1 Mio. peripherer mononukleaerer Zellen (PBMCS) werden jedoch nur noch als grenzwertig gesund eingestuft.

Mehr als 10 maligne Zellen pro 1 Mio. peripherer mononukleaerer Zellen des Blutes sind verdächtig für den frühen Beginn einer Mikrometastasierung eines vorhandenen malignen Tumors, sogar dann, wenn dieses noch nicht mit bildgebenden Verfahren (Röntgen, Onko-PET, Magnetresonanz, Computertomographie) nachweisbar ist.

Treten allerdings 30 und mehr maligne Zellen pro 1 Mio. peripherer mononukleaeren Zellen auf, ist von manifester Mikrometastasierung auszugehen.

Werden jedoch 50 und mehr maligne Zellen pro 1 Mio. peripherer mononukleaeren Zellen und damit Blut-Monozyten nachgewiesen, ist ein primärer Tumor als Ursache dieser Zellen auch mit bildgebenden Verfahren nachweisbar.

Es ist ferner be bekannt (Cancer Immunology and Immunotherapy; Vol. 54; Januar 2003; S. 80-88, inbes. S.84, Spalte 1 eine Anti-Tumor-Immunität von Ratten-Glioma aufgrund von hsp70-Expression durch Hyperthermie zu erreichen. Dabei wird jedoch von einer In-vivo-Behandlung der Ratten ausgegangen, also von einer Ganzkörper-Hyperthermie. Ratten wurden mit diesem hsp 70 immunisiert. Anschließend wurden diese und unbehandelte Ratten mit-T-9-zellen (dem tumor, der das zuvor applizierte hsp 70 produziert hatte) versehen. Ein signifikant reduziertes tumor wachtum konnte in den tumor produzierten-hsp 70-immunisierter ratten im vergleich zu den unimmunisierten ratten detektiert werden.

Wie noch ersichtlich werden wird, kommt die vorliegende Erfindung ohne eine solche Ganzkörper-Hyperthermie bei der In-vitro-Diagnose eines Glioms oder Astrozytoms aus.

Es ist auch schon bekannt (Experimental Cell Research, Vol. 198, 1992, S. 93-100; Japanese Journal of Clinical Medicine, Vol. 53, Nr. 11, 1995, Zusammenfassung), D5 als Proteinmarker im Zusammenhang mit der Diagnose von Glioma- oder Astrozytomazellen c-erb und eine Aberration von p53 bzw. eine Urokinase einzusetzen. Über eine In-vitro-Diagnose eines Glioms oder Astrozytoms eines Individuums unter Heranziehung einer leukapheretisch behandelten Blutprobe des betreffenden Individuums ist in diesem Zusammenhang allerdings nichts bekannt.

Es ist überdies bekannt (Journal of Cellular Biochemistry; Vol. 63, 1996, S. 51-60), dass das Hitze-Schock-Protein hsp66 in Gliomazellen nach einem starken Hitze-schock zusammen mit hsp90 und hsp72 induziert wird. Auch in diesem Zusammenhang ist indessen nichts über eine In-vitro-Diagnose eines Glioms oder Astrozytoms und der Verwendung einer solche Zellen enthaltenden, leukapheretisch behandelten Blutprobe eines Individuums bekannt.

Um zu bestimmen, ob eine Blutprobe Glioma- oder Astrozytomazellen enthält, können verschiedene Methoden bzw. Verfahren angewandt werden. Diese Verfahren basieren, wie aus dem Stand der Technik bekannt, auf spezifischen Eigenschaften besagter Zellen, beispielsweise auf der pathologischen Morphologie, wie sie durch lichtmikroskopische Methoden charakterisiert werden kann, auf dem PCRgestützten Nachweis glioblastomspezifischer mRNA, beispielsweise von GFAP oder auf Fluoreszenz-in-situ-Hybridisierung geschädigter Genome, abgekürzt FISH, oder auf dem Nachweis der Expression bestimmter Onkoproteinmarker, wie sie mit immuncytochemischen Mitteln nachgewiesen werden können.

Die vorstehend erwähnten Methoden bzw. Verfahren liefern jedoch erst in einem relativ späten Stadium der Erkrankung verwertbare Diagnoseaussagen, das heißt dann, wenn Glioma- oder Astrozytomazellen in erheblicher Anzahl, also über 30 Zellen pro 1 Mio. peripherer mononukleaeren vorhanden sind.

Wie oben erwähnt, verbleiben dem jeweiligen Patienten in einem solchen Falle nach Durchführung einer Diagnose, wie sie oben erwähnt worden ist, in der Regel nur noch eine oder mehrere der konventionellen Therapien zur Beseitigung des vorhandenen Tumors oder der vorhandenen Tumoren mit den geschilderten Problemen.

Ganz allgemein besitzen konventionelle Krebstherapien den Nachteil stärkerer Nebenwirkungen für den Patienten; vor allem verursachen sie Schmerzen und eine Reduktion der immunologischen Funktionen des Patienten. Oft sind diese Therapien kaum nützlich und verlängern, wenn überhaupt die Lebenserwartung nur innerhalb sehr enger Grenzen; dies gilt speziell für das Gliom. So blieb die Behandlung und Früherkennung des Glioblastoma-multiforme über Jahrzehnte hinweg enttäuschend. Trotz Anwendung von fortgeschrittenen chirurgischen Techniken und postoperativer Nachbestrahlung sowie Chemotherapie blieb die Rate lebensbedrohlicher Rezidive extrem hoch (80 % der Fälle erlitten innerhalb von zwei Jahren nach einer Operation ein Tumor-Rezidiv, sogar dann, wenn der primäre Tumor komplett entfernt gewesen zu sein schien) (Fine et al., Cancer, Philadelphia 71 (1993), 2585-2597).

Der Erfindung liegt daher die Aufgabe zu Grunde, ein gegenüber den eingangs betrachteten bekannten Verfahren zur Diagnose eines Glioms oder Astrozytoms alternatives Verfahren zur In-vitro-Diagnose solcher Tumoren (Glioma oder Astrozytoma) zu einem möglichst frühen Zeitpunkt anzugeben, um nach deren Diagnose eine wirksame Therapie praktisch zu Beginn der Erkrankung starten zu können und um somit das Leben des jeweils betroffenen Patienten zu erhalten oder dessen Lebenserwartung wenigstens zu verbessern. Anders ausgedrückt soll durch die vorliegende Erfindung für eine Verbesserung der Gliom-Diagnostik in einem sehr frühen Stadium gesorgt werden. Unter einem möglichst bzw. sehr frühen Stadium wird hier ein Stadium verstanden, in dem mit den bisherigen konventionellen Diagnoseverfahren noch keine malignen Zellen nachweisbar sind, also 1 bis 10 maligne Zellen pro 1 Mio. peripherer mononukleaerer Zellen.

Gelöst wird die vorstehende Aufgabe bei einem Verfahren zur In-vitro-Diagnose der eingangs genannten Art erfindungsgemäß durch.

Ausführen folgender Schritte:
- eine von dem betreffenden Individuum entnommene Blutprobe wird leukapheretisch behandelt
- und in der leukapheretisch behandelten Blutprobe des betreffenden Individuums wird die Gegenwart von zirkulierenden Glioma- oder Astrozytomazellen dadurch bestimmt, dass an einen von den genannten Glioma- oder Astrozytomazellen exprimierten Proteinmarker ein Antikörper gebunden wird, durch den die Anwesenheit eines primären Glioms oder Astrozytoms des betreffenden Individuums detektiert wird.

Die Erfindung bringt den Vorteil einer besonders einfachen und sicheren Diagnose des Vorhandenseins von Gliomen oder Astrozytomen in einem Patienten mit sich, indem sie die überraschende Erkenntnis ausnutzt, dass im Falle von Gliomen und Astrozytomen die Blut-Hirn-Barriere des betroffenen Patienten durchlässig, also offen ist und der Tumor in die Blutbahn Glioblastomazellen emittiert, die dann darin zirkulieren. Diese Zellen werden gemäß vorliegender Erfindung als diagnostisches Werkzeug genutzt und dienen darüber hinaus als therapeutische Quelle für die In-vitro-Isolation von Hitze-Schock-Proteinen und Onkogenen sowie Tumor-Antigenen aus disseminierenden Tumorzellen.

Diese Diagnose gemäß der Erfindung ist zu einem sehr frühen Zeitpunkt des Auftretens eines Glioms oder Astrozytoms erfolgreich möglich.

Bei dem Verfahrens gemäß der Erfindung erfolgt der Nachweis der Gegenwart zirkulierender Glioma- oder Astrozytomazellen, basierend auf der Bindung einer Sonde, nämlich eines Antikörpers an einem Protein, das spezifisch von Glioma- oder Astrozytomazellen exprimiert wird. Bevorzugte Proteinmarker dafür sind das gliofibrilläre saure Protein (GFAP), das durch Anwendung eines kommerziell verfügbaren Antikörpers (Sigma Aldrich GmbH, Eschenstr. 5, 82024 Taufkirchen) überprüft werden kann, eine tumorspezifische Protease, vorzugsweise die Urokinase, Onkoproteine, wie c-myc, src, ck-ras, p53 mutant, VEGF, erb/B2, P MDR (multi-drug-resistanceassoziierte Onkoproteine).

Durch diesen molekularen Ansatz können sowohl neue Konsequenzen für die Früherkennung von Glioblastomen und ihren Rezidiven als auch für die therapeutischen Strategien bzw. Verfahren und insbesondere für die Prophylaxe von Rezidiven gezogen werden.

Um die im Zusammenhang mit dem erfindungsgemäßen Verfahren zur In-vitro-Diagnose erwähnte wirksame Therapie bereits zu Beginn der Erkrankung starten zu können, bezieht sich die vorliegende Erfindung außerdem auf eine pharmazeutische Mischung zur Behandlung eines Glioms, Astrozytoms oder eines Rezidivs davon mit wenigstens einem Hitze-Schock-Protein, vorzugsweise hsp70, hsp90, hsps104, hsp60 und/oder hsp27, welches von einem Gliom oder Astrozytom eines Individuums aus einem Glioma- oder Astrozytomazellen enthaltenden-Blutproben-Leukapherisat des betreffenden Individuums exprimiert ist. Dies heißt, dass die pharmazeutische Mischung wenigstens ein Leukapherisat-Hitze-Schock-Protein enthält.

Die Erfindung ermöglicht damit die Anwendung therapeutischer Strategien unter Ausnutzung der vorstehend erwähnten Erkenntnis, dass im Falle des Vorhandenseins von Gliomen und Astrozytomen die Blut-Hirn-Barriere des betreffenden Patienten durchlässig ist, was zur Zirkulation von Glioma- und Astrozytomazellen im Blute des betreffenden Patienten führe.

Die vorliegende Erfindung bezieht sich somit, wie oben ausgeführt, auch auf therapeutische Ansätze mit isolierten Stoffgemischen aus disseminierenden Tumorzellen, basierend auf der Erkenntnis, dass bei Gliomen/Astrozytomen die Blut-Him-Schranke geöffnet ist, was in der Emission zirkulierender transformierter Zellen resultiert.

In diesem Zusammenhang sei hervorgehoben, dass zum ersten Male festgestellt werden konnte, dass autologe Hitze-Schock-Proteine (HSP) und gegebenenfalls Onkoproteine aus im Blutstrom eines Patienten zirkulierenden Glioblastoma-multiforme-Zellen gewonnen, eine stabile Remission bei Patienten mit chemo- und strahlenresistentem Glioblastoma-multiforme erzeugen und dass darüber hinaus eine Verlaufskontrolle der Zahl und des Onkoproteinstatus von zirkulierenden Glioblastomazellen durchführbar ist.

Für die Anwendung der pharmazeutischen Mischung gemäß der Erfindung werden die vorstehend erwähnten Hitze-Schock-Proteine vorzugsweise miteinander und/ oder mit geeigneten pharmazeutischen Trägem kombiniert. Beispiele für geeignete pharmazeutische Träger sind bekannt und umfassen eine phosphatgepufferte Kochsalzlösung, Wasser, Emulsionen, wie Öl-Wasser-Emulsionen, verschiedene Typen von Wachsen, sterile Lösungen, usw.

Solche Träger können durch konventionelle Verfahren hergestellt und in geeigneten Dosen angewandt werden. Die Anwendung geeigneter Mischungen kann auf verschiedenen Wegen erfolgen, beispielsweise intravenös, intraperitoneal, subkutan, intramuskulär, topisch oder auf intradermalem Wege.

Der Weg der Anwendung hängt selbstverständlich von der Art der pharmazeutischen Zusammensetzung ab. Das Dosisregime wird vom verantwortlichen Arzt entsprechend aller verfügbarer klinischer Faktoren bestimmt. Wie in der medizinischen Kunst wohl bekannt, hängen Dosierungen für jeden Patienten von vielen Faktoren einschließlich der Größe, der Körpermasse und der Körperoberfläche, dem Alter und dem Geschlecht des Patienten ab. Dies bestimmt die zeitlichen Abläufe und bevorzugten Wege der Anwendung, ebenso wie Art, Stadium und Größe des Tumors. Die Anwendung wird ebenso von der allgemeinen Gesundheit und von sonstigen angewandten Medikamenten, die konkurrierend angewandt werden, beeinflusst.

Bei einer weiteren bevorzugten Anwendung werden monozytäre Zellen des Patienten mit wenigstens einem Hitze-Schock-Protein, abgeleitet aus zirkulierenden Gliomazellen, beladen. Vorzugsweise handelt es sich dabei um die Heat-Shock-Proteine hsp70, hsp90, hsp104, hsp60 und/oder hsp27.

Die folgenden Beispiele illustrieren die Erfindung

### Beispiel 1

Ermittlung zirkulierender Glioblastomzellen

Bei einem Patienten, der an einem Glioblastoma-multiforme Rezidiv in der Hippocampus-Aera in einer Größe von 40x35x30 mm litt, zu dem es nach chirurgischer Entfernung des Primärtumors und maximalen Strahlen - und Chemotherapie-Dosen mit Temezolomid gekommen war, ohne dass diese Medikation in einer Remission resultierte, konnten durch Anwendung einer Apherese im Leukapherisat unter Gewebekulturbedingungen Glioblastoma-multiforme-Zellen nachgewiesen werden.

Diese Zellen wurden molekular charakterisiert und quantifiziert. Die Ergebnisse sind in Tabelle 1 angegeben.

Onkoproteine, exprimiert durch eine entsprechende Zahl von malignen Zellen bezogen auf jeweils 1 Mio. peripherer Blut-Monozyten (PBMC).

**Tabelle 1**

| | |
|---|---|
| c-myc | 100 |
| src | 38 |
| ck-ras | 59 |
| p53 mutant | 25 |
| VEGF | 42 |
| erb/B2 | 100 |
| GFAP | 50 |
| Urokinase | 68 |

### Beispiel 2

Behandlung eines Glioblastoms durch Immuntherapie

Eine Immuntherapie wurde mit autologen Hitze-Schock-Proteinen durchgeführt, stammend aus den zirkulierenden Glioblastoma-multiforme-Zellen eines Patienten.

### Materialien und Methoden

### a) Kontrolle

Die Entwicklung der Erkrankung des Patienten (Glioblastoma-multiforme-Rezidiv) wurde zusätzlich kontrolliert durch konventionelle bildgebende Verfahren, hier: Kernspinresonanz, neurologische Expertenmeinung und Quantifizierung sowie durch molekulare Charakterisierung der Zahl und des Onkoprotein-Expressions-Status der zirkulierenden Glioblastoma-multiforme-Zellen.

### b) Zellkultur

Weiße periphere, mononukleaere Zellen des Patienten wurden durch Leukapherese erhalten.

Ganz generell gilt nach dem aus der eingangs zitierten EP 0 584 715 B1 folgendes.

Während einer Apherese wird die Komponente mit niedrigerer Dichte aus dem Volumen durch Abtrennung der Zellen des Vollblutes vom Blutplasma in einer Zentrifuge mit einem Fraktionierungsvolumen zum Trennen von Vollblut in Blutkomponenten abgeteilt, wobei jede davon eine spezifische Dichte hat, so dass jede der Blutkomponenten eine Region mit einer radialen Position in dem Fraktionierungsvolumen gemäß ihrer Dichte besetzt. Dabei schließen die Blutkomponenten eine Komponente mit niedrigerer Dichte und Komponenten mit höherer Dichte ein. Die Komponenten mit höherer Dichte sind rote Blutzellen (Erythrozyten), weiße Blutzellen (Leukozyten) beinhaltend die Monozyten gegebenenfalls disseminierende Tumorzellen und Thrombozyten. Die Komponente mit niedrigerer Dichte ist das Blutplasma. Während der Apherese wird die Komponente mit niedrigerer Dichte aus dem Volumen durch eine Auslassöffnung in einen ersten Behälter verdrängt. Die Blutpumpe wird gestoppt und die gesammelte Komponente mit niedrigerer Dichte wird dann zu dem Trennvolumen durch die Einlaßöffnung mit einer zweiten Fließgeschwindigkeit zugeführt. Die zweite Fließgeschwindigkeit ist größer als der erste Fluß durch eine "Leukozytenmanschette", bestehend aus Leukozyten, Monozyten, gegebenenfalls Tumorzellen und Thrombozyten, wodurch die Leukozytenmanschette verdünnt und erweitert wird. Die erweiterte Leukozytenmanschette verbessert die Trennung zwischen Leukozyten, Monozyten und Thrombozyten indem den dichteren weißen Blutzellen erlaubt wird, von den leichteren Thrombozyten vollständiger zu den äußeren Schichten der Leukozytenmanschette zu sedimentieren. Auf diese Weise wird die Trennung der Komponenten mit höherer Dichte, die in dem Volumen zurückbleiben, verbessert.

Diese Leukozytenmanschette, enthaltend Leukozyten, Monozyten und gegebenenfalls Tumorzellen wird dann als Leukapherisat über einen Transportbeutel in eine Zellkultur übergeführt.

Das in Zellkultur überführte Leukapherisat wird bezüglich der Kontamination maligner transformierter Zellen durch Zählen solcher Zellen überprüft:
10 und mehr maligne Zellen pro 1 Mio. peripherer mononuklearer Zellen des Blutes sind verdächtig für den frühen Beginn einer Mikrometastasierung des malignen Tumors, selbst wenn dieser noch nicht mit bildgebenden Verfahren nachweisbar ist.
30 und mehr maligne Zellen pro 1 Mio. peripherer Monozyten des Blutes machen eine Mikrometastasierung nachweisbar.
50 und mehr maligne Zellen pro 1 Mio. peripherer Blut-Monozyten machen den primären Tumor auch durch bildgebende Verfahren nachweisbar.

Die Zellen wurden aus dem Leukapherisat in 96-Wellplatten unter Zellkultur-Bedingungen überführt und dort mittels monoklonaler Antikörper immuncytochemisch bezüglich der Expression von c-myc, src, ck-ras, p53 mutant, VEGF, erb/B2, Urokinase, GFAP und MDR untersucht.

Die polymorphonukleaeren Zellen des Leukapherisates wurden in Tumor-Antigen präsentierende dendritische Zellen expandiert durch Zusatz von GMCSF (800 E pro ml) und Interleukin-4 (250 E pro ml), vertrieben durch Chiron GmbH, Linprunstr. 16, 80335 München in X-Vivo 15 Medien vertrieben durch Cambrex BioSciences, B-4800 Verviers, Belgien.

Die Tumorzellen, die gemäß Apherese wie unter b) beschrieben, gesammelt worden waren, wurden für zwei Wochen in MEM-Medium, vertrieben durch Sigma Aldrich GmbH, Eschenstr. 5, 82024 Taufkirchen, und autologem Plasma kultiviert.

Dann wurden diese Zellen über Nacht noch einmal bei 42°C kultiviert. Danach wurden die Tumorzellen gesammelt und durch 4-5 Einfrier-/Auftauzyklen lysiert. Große Partikel wurden durch Zentrifugation entfernt, der Überstand wurde durch einen 0,2-Am-Filter gepreßt und Aliquots wurden bei -80°C bis zum Gebrauch eingefroren. Das für die Beladung (Pulsung) der dendritischen Zellen verwendete Aliquot enthielt hsp 70, 90, 104, 60, 27, grp 58, 94 sowie Telomere (Ashley et al, J. Exp. Med. 186 (1997), 1177-1182)

Ergebnisse:
Pathophysiologische Konsequenzen:
   Durch die infiltrierende Wirkung der Proteasen der Tumorzelle und deren Sekretion von VEGF (vaskularer endothelialer Wachstumsfaktor) wurde der Tumor offensichtlich invasiv.

Als Ergebnis des Vorhandenseins von MDR (multi-drug-resistance assoziierten Proteinen) sprach er nicht auf eine Strahlen- oder Chemotherapie an.

### Tabelle 2

Onkoproteine, exprimiert von der Zahl der malignen Zellen pro 1 Mio. peripherer Blut-Monozyten

| | |
|---|---|
| c-myc | 20 |
| src | 4 |
| ck-ras | 18 |
| p53 mutant | 9 |
| VEGF | 11 |
| erb/B2 | 28 |
| MDR | 10 |
| GFAP | 15 |
| Urokinase | 12 |

Zum ersten Mal konnte somit demonstriert werden, dass
a) die Blut-Hirn-Barriere bei Patienten mit Glioblastoma-multiforme offen ist und der Tumor zirkulierende Glioblastomazellen in die Blutbahn freisetzt, die für diagnostische und auch für die Herstellung pharmazeutischer bzw. therapeutischer Stoffgemische genützt werden können.
   und
b) eine Vaccinierung mit autologen Hitze-Schock-Proteinen, gewonnen aus den zirkulierenden Tumorzellen und Onkoproteinen, effektiv ist.

## Patentansprüche

1. Verfahren zur In-vitro-Diagnose eines Glioms oder Astrozytoms eines Individuums als Glioblastoma-multiforme durch Ausführen folgender Schritte:
• eine von dem betreffenden Individuum entnommene Blutprobe wird leukapheretisch behandelt
• und in der leukapheretisch behandelten Blutprobe des betreffenden Individuums wird die Gegenwart von zirkulierenden Glioma- oder Astrozytomazellen **dadurch** bestimmt, dass an einen von den genannten Glioma- oder Astrozytomazellen exprimierten Proteinmarker ein Antikörper gebunden wird, durch den die Anwesenheit eines primären Glioms oder Astrozytoms des betreffenden Individuums detektiert wird.

2. Verfahren nach Anspruch 1, **dadurch gekennzeichnet, dass** als Proteinmarker ein gliafibrilläres saures Protein (GFAP) eingesetzt wird.

3. Verfahren nach Anspruch 1, **dadurch gekennzeichnet, dass** als Proteinmarker eine Protease, c-myc, src, ck-ras, p53 m, VEGF, erb/B2, MDR eingesetzt wird.

4. Verfahren nach Anspruch 3, **dadurch gekennzeichnet, dass** als Protease eine Urokinase eingesetzt wird.

## Claims

1. Process of diagnosing in vitro a glioma or an astrocytoma of an individual as glioblastoma multiforme by performing the following steps:
• subjecting a blood sample taken from the individual in question to leukapheresis,
• and determining the presence of circulating glioma or astrocytoma cells in the blood sample subjected to leukapheresis by binding to a protein marker expressed by said glioma or astrocytoma cells an antibody by which the presence of a primary glioma or astrocytoma of the individual in question is detected.

2. Process according to Claim 1, **characterized in that** the protein marker used is a glial fibrillary acidic protein (GFAP).

3. Process according to Claim 1, **characterized in that** the protein marker used is a protease, c-myc, src, ck-ras, p53m, VEGF, erb/B2, MDR.

4. Process according to Claim 3, **characterized in that** the protease used is a urokinase.

## Revendications

1. Procédé de diagnostic in vitro d'un gliome ou d'un astrocytome d'un individu comme étant un glioblastome multiforme, par mise en oeuvre des étapes suivantes :
- on traite par leucaphérèse un échantillon sanguin prélevé de l'individu concerné,
- et, dans l'échantillon sanguin traité par leucaphérèse de l'individu concerné, on détermine la présence de cellules circulantes de gliome ou d'astrocytome, par le fait qu'à un marqueur protéique exprimé par lesdites cellules de gliome et d'astrocytome est lié un anticorps, grâce auquel on détecte la présence d'un gliome ou d'un astrocytome primaire de l'individu concerne.

2. Procédé selon la revendication 1, **caractérisé en ce qu'**on utilise en tant que marqueur protéique une protéine acide gliofibrillaire (GFAP).

3. Procédé selon la revendication 1, **caractérisé en ce qu'**on utilise en tant que marqueur protéique une protéase, c-myc, src, ck-ras, p53m, VEGF, erb/B2, MDR.

4. Procédé selon la revendication 3, **caractérisé en ce qu'**on utilise en tant que protéase une urokinase.
